# EUROPÄISCHE PATENTANMELDUNG

(11) **EP 4 467 072 A1**
(43) Veröffentlichungstag der Anmeldung: **27.11.2024**
(21) Anmeldenummer: 24178157.4
(22) Anmeldetag: 27.05.2024
(51) Int. Cl.: A61B 5/083, A61B 5/087, A61B 5/097, A61B 5/22, G01N 21/64, G01N 33/00, G01N 33/497

(54) **ERGOSPIROMETRIEVORRICHTUNG UND ENTSPRECHENDES HERSTELLUNGSVERFAHREN**

(30) Priorität: 25.05.2023 DE 102023204890
(71) Anmelder: KNESTEL Technologie & Elektronik GmbH, 87496 Hopferbach (DE)
(72) Erfinder: KNESTEL, Markus, 87496 Hopferbach (DE)
(74) Vertreter: MERH-IP Matias Erny Reichl Hoffmann Patentanwälte PartG mbB

(57) **Zusammenfassung**

Die vorliegende Erfindung betrifft eine Ergospirometrievorrichtung zur Erfassung von Parametern eines Atemgases, mit einem Grundkörper 1, der eine Atemmaske oder ein Mundstück aufweist und einen Atemgasführungsabschnitt 5 aufweist, und einer Messeinrichtung 4 zum Erfassen von Parametern des Atemgases, die eine Recheneinheit 3 zum Verarbeiten der erfassten Parameter des Atemgases aufweist, wobei die Parameter des Atemgases zumindest eine Sauerstoffkonzentration und/oder eine Kohlenstoffdioxidkonzentration im Atemgas umfassen, wobei die Messeinrichtung 4 zumindest einen optischen Sauerstoffsensor 10 zum Erfassen der Sauerstoffkonzentration des Atemgases und/oder einen optischen Kohlenstoffdioxidsensor aufweist und wobei das Erfassen der Sauerstoffkonzentration auf einem Prinzip der Lumineszenzlöschung und/oder einem Prinzip der dualen Emission beruht.

## Beschreibung

Die vorliegende Erfindung betrifft eine Ergospirometrievorrichtung zur Erfassung von Parametern eines Atemgases wie beispielsweise die Sauerstoffkonzentration (insbesondere die Disauerstoffkonzentration/O₂-Konzentration/Konzentration molekularen Sauerstoffs) und/oder die Kohlendioxidkonzentration (CO₂-Konzentration) und ein entsprechendes Herstellungsverfahren.

### Hintergrund

Eine alternative Bezeichnung für Ergospirometrie ist Spiroergometrie. Bei der Ergospirometrie (von έργov ("ergon") Altgriechisch für "Arbeit" oder "Werk", spirare Latein für "atmen" und µέτρov ("metron") Altgriechisch für "Maß") werden Atemgase bei körperlicher Belastung gemessen. Insbesondere kann ein Atemminutenvolumen (oder allgemeiner ein Atemzeitvolumen) gemessen werden. Zudem können die Kohlendioxid- und/oder die (Di-)Sauerstoffkonzentration im Atemgas gemessen werden.

Mobile Ergospirometriegeräte, z. B. für Belastungsuntersuchungen beim Menschen, sind seit einigen Jahren bekannt. Mithilfe derartiger Systeme lassen sich Analysen direkt auf dem Sport- oder Arbeitsplatz unter natürlichen Bedingungen und Belastungssituationen durchführen. Über Telemetrieeinheiten werden Messdaten in Echtzeit an einen Personal Computer oder ein Notebook übertragen, wobei eine entsprechende Steuerung des Trainings- oder Übungsverlaufes nach Auswertung der Daten am Personal Computer möglich ist. Durch derartige Geräte wurden neue Anwendungsgebiete in der Leistungsdiagnostik in der Arbeits-, Sport- und Rehabilitationsmedizin erschlossen.

Ein Ergospirometriesystem ist beispielsweise aus der DE 199 60 257 C1 bekannt. Insbesondere wird ein Ergospirometriesystem für Tiere, wie Kamele oder Pferde, beschrieben. Über trichterförmige oder zylindrische Atemgasmasken und mit Gasvolumenstrom- oder Mengensensoren, sowie eine Messeinheit mit Sensoren zur Bestimmung der Kohlendioxid- oder Disauerstoffkonzentration im Atemgas, werden die Werte nach dem Mischkammer- oder dem Breath-by-Breath-Prinizip ermittelt und im Anschluss über Signalübertragungsmittel zur Messwertweiterverarbeitung, Darstellung und Analyse zu einer Basisstation übermittelt. Zur Bestimmung des Volumenstroms sind Ultraschallwandler vorgesehen.

Auch die DE 199 53 866 B4 betrifft ein mobiles Ergospirometriesystem mit einer Messeinheit, welche am Probanden fixierbar ist und Gasvolumen- sowie Mengensensoren zur Bestimmung der Kohlendioxid-/Disauerstoffkonzentration im Atemgas umfasst. Über einen Signalverarbeitungsprozessor und ein Telemetriemodul sowie eine computergestützte Basisstation mit Telemetrieeinheit zum Aufbau einer drahtlosen Verbindung mit dem Telemetriemodul können von der Basisstation aus online mit dem Probanden Informationen oder Aufforderungen zur Bedienung der Messeinheit und/oder zur Gestaltung des Versuchslaufs übermittelt werden.

Die DE 10 2016 214 702 A1 beschreibt eine Vorrichtung zur Erfassung von Parametern eines Atemgases, die einen Energiespeicher aufweist.

Ergospirometrievorrichtungen arbeiten oft in einem sogenannten Mischkammerprinzip, d. h. diese Vorrichtungen sammeln die ausgeatmete Atemluft mehrerer Atemzüge in einer Kammer und führen die Atemgasmessung in dieser Kammer durch. Moderne Ergospirometrievorrichtungen hingegen bestimmen die Atemgasparameter in einem Breath-by-Breath-Prinzip, d. h. diese Ergospirometrievorrichtungen können die Atemgasparameter für jeden Atemstoß bestimmen.

Eine Aufgabe der vorliegenden Erfindung ist es, eine verbesserte Ergospirometrievorrichtung bereitzustellen, die insbesondere für die Parameter des Atemgases, insbesondere die Konzentration von Sauerstoff und/oder der Konzentration von Kohlendioxid eine deutlich höhere Empfindlichkeit und eine hohe Geschwindigkeit der Auswertung aufweist.

Zur Erfüllung dieses Bedarfs wird vorliegend eine Ergospirometrievorrichtung zur Erfassung von Parametern eines Atemgases vorgeschlagen. Die Ergospirometrievorrichtung kann dabei aufweisen: einen Grundkörper, der eine Atemmaske oder ein Mundstück aufweist und einen Atemgasführungsabschnitt aufweist, und eine Messeinrichtung zum Erfassen von Parametern des Atemgases, die eine Recheneinheit zum Verarbeiten der erfassten Parameter des Atemgases aufweisen kann, wobei die Parameter des Atemgases zumindest eine Sauerstoffkonzentration und/oder eine Kohlenstoffdioxidkonzentration im Atemgas umfassen kann. Die Messeinrichtung kann zumindest einen optischen Sauerstoffsensor zum Erfassen der Sauerstoffkonzentration des Atemgases und/oder einen optischen Kohlenstoffdioxidsensor (bzw. Infrarot-Kohlenstoffdioxidsensor) aufweisen und das Erfassen der Sauerstoffkonzentration kann auf einem Prinzip der Lumineszenzlöschung und/oder einem Prinzip der dualen Emission beruhen.

Vorzugsweise kann die Messeinrichtung dazu ausgebildet sein, die Erfassung der Sauerstoffkonzentration über eine Lebensdauerauswertung (bspw. der Lumineszenz bzw. physikalischen Lebensdauer einer Lumineszenzabklingkurve) vorzunehmen, durch Zuordnung der gemessenen Lebensdauer zur Sauerstoffkonzentration. Es kann beispielsweise mit einem kurzen Anregungspuls angeregt werden und die Lumineszenzabklingkurve kann beobachtet werden. Beispielsweise kann bei einem einfach exponentiellen Abfall die Lebensdauer aus zwei Intensitäten, die zu den Zeitpunkten t1 und t2 während des Abfalls detektiert werden, bestimmt werden. Bei lumineszierenden Stoffen, deren Lebensdauer in Abhängigkeit der Konzentration eines Quenchers gequencht wird, kann die Lebensdauer mit Hilfe der RLD-Methode bestimmt werden. Das Verfahren Rapid-Lifetime-Determination (RLD) ermöglicht eine schnelle Bestimmung der Lebensdauer von Fluoreszenzsignalen durch Auswertung der Änderung des Fluoreszenzsignals nach einem kurzen Anregungsimpuls, typischerweise 10 bis 100 ns. Die Lebensdauer der Fluoreszenz wird durch eine Exponentialfunktion beschrieben und kann durch eine schnelle Datenaufnahme und -verarbeitung in Echtzeit bestimmt werden. Alternativ (oder auch zusätzlich) kann die Lebensdauer durch Phasenmodulation bestimmt werden. Bei der Phasenmodulation wird die Lebensdauer über den Tangens der Phasenverschiebung dividiert durch die Anregungsfrequenz bestimmt. Alternativ (oder auch zusätzlich) kann die Lebensdauer durch eine Detektion der Zefallsdynamik bestimmt werden.

Ist für ein System die Lebensdauer in Abhängigkeit der Quencherkonzentration bekannt, so kann der gemessenen Lebensdauer die Quencherkonzentration zugeordnet werden. Dieses Prinzip kann auch zur pixelweisen Berechnung der Lebensdauer genutzt werden, bspw. mittels eines (oder mehrerer) CCD-Flächensensors, und es kann pixelweise eine Konzentration molekularen Sauerstoffs bestimmt werden.

Vorzugsweise kann die Messeinrichtung dazu ausgebildet sein, die Lebensdauerauswertung über eine Detektion der Zerfallsdynamik, über eine schnelle Lebensdauerbestimmung, RLD, "Rapid Lifetime Determination", und/oder über eine Phasenmodulation vorzunehmen.

Vorzugsweise kann die Messeinrichtung dazu ausgebildet sein, die Erfassung der Sauerstoffkonzentration über eine Auswertung der Quantenausbeute und/oder der Intensität vorzunehmen.

Vorzugsweise kann die Messeinrichtung zur ratiometrischen Bestimmung der Sauerstoffkonzentration eingerichtet sein und aus einem Intensitätsverhältnis der Fluoreszenz und Phosphoreszenz die Sauerstoffkonzentration bestimmen.

Vorzugsweise kann der Sauerstoffsensor auf dem Prinzip der Lumineszenzlöschung mit Metall-Komplexen (insbesondere Übergangsmetallkomplexe) arbeiten, wobei die Übergangsmetall-Komplexe bevorzugt Platin und/oder Kupfer umfassen können. Bevorzugt erfolgt die Anregung mit einer schmalbandigen LED, wobei der Photodetektor mit einfacher Optik bei geringem Abstand vorgesehen ist. Bei größerem Abstand können Linsensysteme verwendet werden. Bei Störlicht aus Umgebung kann zudem ein Bandpassfilter verwendet werden.

Vorzugsweise kann über Variation von Metall-Komplex und Anregungsenergie eine Duale Emission möglich sein, bevorzugt in den Bereichen 375 - 445 nm und 450 - 650 nm, um Informationen über Druck und/oder Temperatur abzuleiten.

Vorzugsweise kann der Sensor auch über Feststoffe funktionieren, die nicht fluoreszierend sind und die transparent sind, insbesondere Glas, PC (Polycarbonat), PMMA (Polymethylmethacrylat, auch Acrylglas genannt), PET (Polyethylenterephthalat). Dies ermöglicht ein sehr geringes Messvolumen. Ein kleines Messvolumen hat den Vorteil, dass die Atemluft schnell ausgetauscht werden kann und dass der Atmung mit nur wenig Zeitverzug (Totzeit) und hoher zeitlicher Auflösung der Messwerte gefolgt werden kann. Ein kleines Messvolumen stellt andererseits eine Herausforderung für das Sensordesign dar, da auf dem geringen Volumen die Sensorkomponenten und elektronischen Bauteile untergebracht werden müssen. Mit der vorliegenden Ergospirometrievorrichtung reicht ein Messvolumen von Bruchteilen von einem Milliliter (ml) bis wenige Milliliter für eine korrekte Messung. Vorzugsweise kann das Messvolumen kleiner gleich 0,6 Milliliter betragen, insbesondere zwischen 0,4 und 0,6 Milliliter, insbesondere ungefähr 0,5 Milliliter. Das Messvolumen kann auch kleiner als 0,5 Milliliter sein.

Vorzugsweise kann der Sauerstoffsensor temperatur- und barometrisch kompensiert sein. Beispielsweise kann eine duale Emission mit zwei unterschiedlichen Wellenlängen gemessen werden (2 angeregte Zustände). Die Messeinrichtung kann aber auch zusätzlich einen integrierten Drucksensor umfassen. Zusätzlich oder alternativ dazu kann der Druck auch aus optischen Messungen abgeleitet werden.

Vorzugsweise kann eine Sensorschicht des Sauerstoffsensors eine Diffusionskonstante derart aufweisen, dass die Aufnahme eines Messwertes mit einer Zeit von geringer als 5 Sekunden, und besonders bevorzugt geringer als 0,1 s möglich wird. Bevorzugt wird eine Dynamik des Sensors über eine Zeit t10-t90 bestimmt, d. h. über die Zeit, die ein Sensorsignal benötigt, um von 10% auf 90% des Endwertes anzusteigen. Dies hat den Vorteil, dass so Schmutzeffekte von Zuleitungen und Ähnlichem ausgeschlossen werden.

Vorzugsweise kann ein Trägermaterial der Sensorschicht eine transparente Polyesterschicht oder Polyesterfolie umfassen, welche bevorzugt eine Schichtdicke von weniger als 800 µm aufweist, und wobei die Sensorschicht bevorzugt ein Sensorspot zur kontaktlosen Messung sein kann. Es ist jedoch nicht zwingend ein Trägermaterial erforderlich. Dann kann man ein Metallkomplex direkt (auf die Innenseite des Atemgasführungsabschnitts) auftragen. Vorzugsweise weist die Messeinrichtung einen Sensorspot auf.

Vorzugsweise kann ein Anregungslicht der Anregungsdiode des Sauerstoffsensors blinken. Vorzugsweise kann ein Anregungslicht der Anregungsdiode des Sauerstoffsensors eine Anregungsfrequenz von mehr als 1 Hz, besonders vorzugsweise zwischen 10 Hz und 100 kHz, aufweisen. Vorzugsweise kann das von der Sensorschicht, insbesondere dem Sensorspot, reflektierte Licht im Bereich von 450 - 650 nm und/oder 375 - 445 nm liegen.

Vorzugsweise kann die Messeinrichtung am Grundkörper bereitgestellt sein, damit die Parameter direkt in einem Atemgasstrom erfassbar sind, der durch den Grundkörper geführt wird. Insbesondere ist eine Erfassung der Parameter des Atemgases in situ und/oder in Extraktion möglich.

Vorzugsweise können zumindest Teile der Messeinrichtung von dem Grundkörper beabstandet sein, sodass eine Erfassung der Parameter des Atemgases in Extraktion ermöglicht wird.

Vorzugsweise kann der Kohlenstoffdioxidsensor zum Erfassen einer Kohlendioxidkonzentration im Atemgas vorgesehen sein, wobei das Erfassen der Kohlendioxidkonzentration im Atemgas auf einem Erfassungsprinzip eines nichtdispersiven Infrarotsensors, NDIR-Prinzip, basieren kann.

Vorzugsweise kann der Kohlenstoffdioxidsensor einen Photodetektor und eine Leuchtdiode, insbesondere MIR-LED, für einen Wellenlängenbereich im mittleren Infrarotbereich aufweisen. Insbesondere kann dieser Wellenlängenbereich zwischen 3 µm und 8 µm und besonders vorzugsweise zwischen 4,1 µm und 4,4 µm liegen.

Vorzugsweise kann die Leuchtdiode für einen Wellenlängenbereich im mittleren Infrarot derart modulierbar sein, dass Taktfrequenzen von mindestens 100 kHz erreicht werden.

Vorzugsweise kann der Photodetektor ein Quantentopf-Infrarot-Photodetektor (Quantum Well Infrared Photodetector, QWIP) sein.

Vorzugsweise kann die Leuchtdiode eine MIR-LED mit einem Reflektor aufweisen, so dass eine sehr hohe Strahlqualität erzielt wird.

Vorzugsweise kann der Photodetektor zweikanalig ausführt sein, so dass eine Auswertung eines Referenzsignals zur Überwachung des Strahlers ermöglicht wird.

Vorzugsweise kann der Grundkörper den Atemgasführungsabschnitt zur Führung des Atemgasstroms mit einem probandenseitigen Atemgaseinlass und einem Atemgasauslass aufweisen und der Sauerstoffsensor im Atemgasführungsabschnitt vorgesehen sein.

Vorzugsweise kann die Ergospirometrievorrichtung eine mobile Ergospirometrievorrichtung sein.

Vorzugsweise kann die Ergospirometrievorrichtung zumindest in Teilen, vorzugsweise in Gänze auf Kondensation resistent sein, so dass die Ergospirometrievorrichtung fähig ist, die Parameter des Atemgases auch in Flüssigkeiten zu erfassen.

Vorzugsweise kann die Messeinrichtung eine Empfindlichkeit bis wenige ppb aufweisen und/oder einen sehr hohen Dynamikumfang bis 100%, bis zu 9 Größenordnungen aufweisen.

Vorzugsweise kann die Sensorvorrichtung selbstreferenzierend sein.

Vorzugsweise kann die Messeinrichtung eine Ansprechzeit von kleiner oder gleich 0,1 s aufweisen.

Zur Erfüllung des oben beschriebenen Bedarfs wird vorliegend zudem ein Herstellungsverfahren einer Ergospirometrievorrichtung zur Erfassung von Parametern eines Atemgases beschreiben, wobei das Verfahren folgende Schritte aufweist: Bereitstellen einer Messeinrichtung zum Erfassen von Parametern des Atemgases, die eine Recheneinheit zum Verarbeiten der erfassten Parameter des Atemgases aufweist, Bereitstellen eines Grundkörpers, der eine Atemmaske oder ein Mundstück aufweist, und Verbinden der Messeinrichtung mit dem Grundkörper. Die Messeinrichtung und der Grundkörper können dabei wie oben ausgeführt ausgestaltet sein.

### Kurzbeschreibung der Zeichnungen

Figur 1 zeigt einen schematischen Aufbau eines Sauerstoffsensors einer erfindungsgemäßen Ergospirometrievorrichtung.
Figur 2 zeigt eine Emissionsintensität eines Kupfer-Metallkomplexes in Abhängigkeit der Wellenlänge bei entsprechender Anregung.
Figur 3 zeigt die Abhängigkeit der Lebensdauer von der Sauerstoffkonzentration bei verschiedenen Temperaturen.
Figuren 4a und 4b zeigen schematisch wie verschiedene Sauerstoffkonzentrationen auf das Verhältnis von Fluoreszenz zu Phosphoreszenz Einfluss nimmt.
Figur 5a und 5b zeigen wie verschiedene Temperaturen auf das Lumineszenzverhalten Einfluss nimmt.
Figur 6 zeigt den in Figur 5b gezeigten Temperatureinfluss auf einen grünen und einen blauen Kanal.
Figur 7 zeigt die Empfindlichkeit der Sauerstoffsensorik.
Figur 8 zeigt einen schematischen Aufbau eines Kohlenstoffdioxidsensors.
Figur 9 zeigt schematisch ein knapp gehaltenes pneumatisches Konzept des Gesamtsystems.
Figur 10 zeigt eine perspektivische Darstellung einer Messeinrichtung der Ergospirometrievorrichtung.
Figur 11 zeigt die Messeinheit der Figur 9 in einer Schnittdarstellung.
Figur 12 zeigt einen schematischen Aufbau einer Ergospirometrievorrichtung.
Figur 13 zeigt einen vorteilhaften Aufbau einer erfindungsgemäßen Ergospirometrievorrichtung.

### Ausführliche Beschreibung

Die vorliegende Erfindung betrifft eine Ergospirometrievorrichtung, die Parameter eines Atemgases erfasst.

Ein erfinderischer Aspekt dieser Erfindung ist es, eine Sensorik für Sauerstoff, insbesondere Disauerstoff, und Kohlendioxid mit Sensorprinzipien zu entwickeln, die eine Ansprechzeit von <=0,1 s ermöglicht.

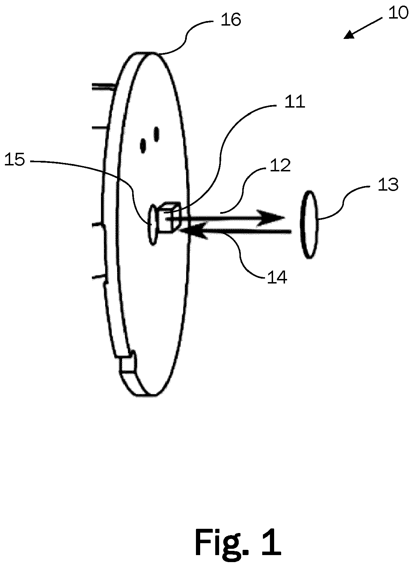

Die Figur 1 zeigt einen schematischen Aufbau eines Sauerstoffsensors 10 einer erfindungsgemäßen Ergospirometrievorrichtung. Eine Lichtquelle 11, die vorzugsweise eine Leuchtdiode (auch LED genannt) 11 ist, sendet einen Anregungsstrahl 12 aus. Der Anregungsstrahl 12 trifft auf einen Sensorspot 13, wo der Strahl "reflektiert" wird. Genauer wird der Strahl absorbiert und wieder emittiert (ausgesendet). Dieses Emissionslicht 14 entspricht einer Lumineszenz. Diese kann eine Fluoreszenz oder eine Phosphoreszenz betreffen. Das Emissionslicht 14 wird schließlich von einem Sensorelement 15 detektiert. Die Leuchtdiode 11 und/oder das Sensorelement 15 können auf einem Träger 16, z. B. einer Leiterplatte, befestigt sein. Das Sensorelement 15 kann ein Photodetektor sein. Alternativ kann das Sensorelement 15 ein Flächensensor (oder mehrere Flächensensoren) sein, wie z. B. eine CCD. Das Vorhandensein einer CCD (oder der einer Vielzahl von Flächensensoren) hat den Vorteil, dass eine räumliche Auflösung der Sauerstoffkonzentration eruiert werden kann.

Der Sauerstoffsensor arbeitet nach dem Prinzip der Lumineszenzlöschung (auch Quenching genannt, z. B. Fluoreszenzlöschung oder Phosphoreszenzlöschung) mit Metall-Komplexen wie z. B. Platin oder Kupfer. Die sensitive Schicht kann sehr dünn aufgebracht werden, so dass sehr kurze Diffusionskonstanten realisierbar sind. Das Messprinzip funktioniert wie folgt: Es erfolgt eine Anregung mit Wellenlänge X und Antwort des Sensor-Spots mit Wellenlänge Y. Durch die geringe Diffusionskonstante von wenigen ms bis µs ist die Ansprechzeit des Spots sehr schnell. Die Auswertung erfolgt optisch über Licht - ebenfalls sehr schnell.

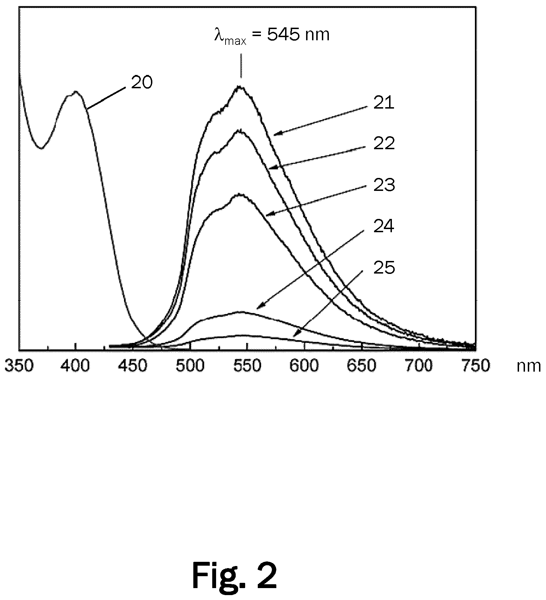

Die Figur 2 zeigt eine Emissionsintensität eines Kupfer-Metallkomplexes in Abhängigkeit der Wellenlänge bei entsprechender Anregung. Als Intensität wird in der Regel eine Flächenleistungsdichte bezeichnet. Sie wird entsprechend (in der Regel) in Watt pro Quadratmeter W/m² gemessen. Die Kurve 20 betrifft vorliegend eine Kurve des Anregungslichts. Die Kurve 21 entspricht einer Emission bei einem Luftdruck von 0,16 Pa (nicht Hektopascal). Die Kurve 22 entspricht einer Emission bei 0,18 hPa (Hektopascal). Die Kurve 23 entspricht einer Emission bei 1,2 hPa. Die Kurve 24 entspricht einer Emission bei 20 hPa. Schließlich entspricht die Kurve 25 einer Emission bei 300 hPa. Wie ersichtlich ist, liegt die die Emissionsspitze bei einer Wellenlänge von 545 nm. Wie auch ersichtlich ist, wird die Emissionsintensität bei zunehmendem Luftdruck immer schwächer. Die Messungen erfolgten bei Raumtemperatur. Die Quantenausbeute für Stickstoff kann hierbei bei 80% liegen und die Lebensdauer bei 1.3 ms. Diese Lebensdauer ermöglicht neben dem Abtasten auch noch eine Signalvorverarbeitung.

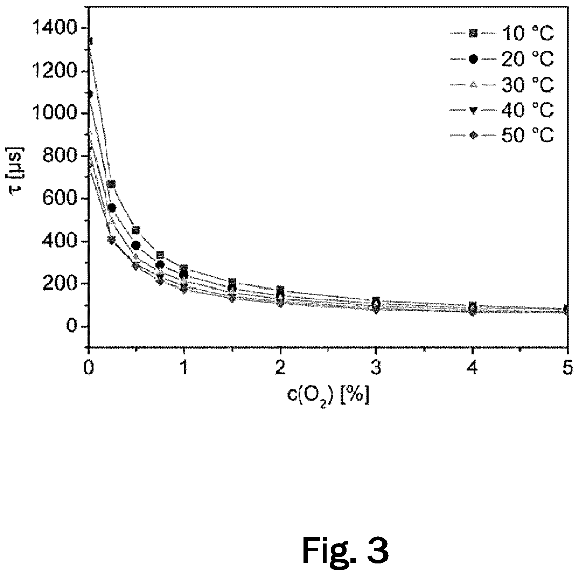

Die Figur 3 zeigt die Abhängigkeit der Lebensdauer von der Sauerstoffkonzentration bei verschiedenen Temperaturen. Wie ersichtlich ist, nimmt bei absolut geringer Sauerstoffkonzentration (<1%) mit steigender Sauerstoffkonzentration die Lebensdauer schnell ab. Zudem nimmt die Lebensdauer mit höherer Temperatur ab.

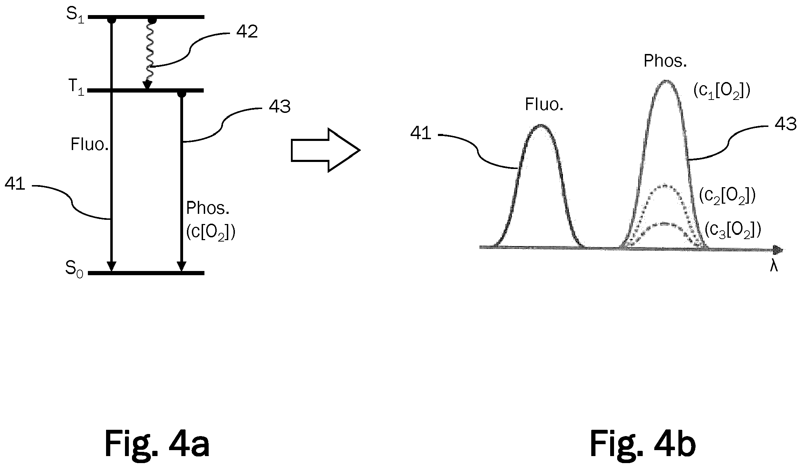

Die Figuren 4a und 4b zeigen schematisch wie verschiedene Sauerstoffkonzentrationen auf das Verhältnis von Fluoreszenz zu Phosphoreszenz Einfluss nimmt. Mit ausreichender Energie zur Anregung lässt sich eine duale Emission (Fluoreszenz und Phosphoreszenz) erzeugen. In Fig. 4a werden die Fluoreszenz und die Phosphoreszenz kurz dargestellt. Ein Elektron wird von einem Grundzustand S0 erst in einen angeregten Zustand versetzt. Ist der angeregte Zustand ein Singulett 1 (S1) fällt in der Regel nach sehr kurzer Zeit (Größenordnung ns) das Elektron wieder in den Grundzustand S0 zurück und emittiert dabei ein Photon. Das ist Fluoreszenz 41. Alternativ dazu kann das Elektron auch in den Zustand Triplett 1 fallen (mit Spinumkehr). Es ist für das Elektron wegen der Spinumkehr 42 physikalisch nicht möglich ohne weiterer Spinumkehr wieder in den Grundzustand zu kommen. Daher braucht es deutlich länger, bis es wieder in diesen Zustand zurückfällt (Größenordnung µs oder länger) (eben mit der weiteren Spinumkehr). Zudem ist das Energieniveau des Triplett 1 tiefer als desjenigen des Singulett 1, so dass das emittierte Photon weniger Energie aufweist, was einer längeren Wellenlänge entspricht. Das ist die Phosphoreszenz. Wie in Fig. 4b gezeigt, wird die Phoshoreszenz (grüne Emission) mit zunehmender Sauerstoffkonzentration gelöscht. Es ist dabei zu sagen, dass die Sauerstoffkonzentration bei es die größte der dreien ist, bei c₂ die mittlere der dreien und bei c₁ die geringste der dreien. Die Temperatur ist hierfür konstant gehalten. Es sei hier auch betont, dass bei zunehmender Sauerstoffkonzentration explizit die Phosphoreszenz gelöscht wird. Die Fluoreszenz bleibt davon unberührt. Damit ist dieses System / dieses Prinzip selbstreferenzierend.

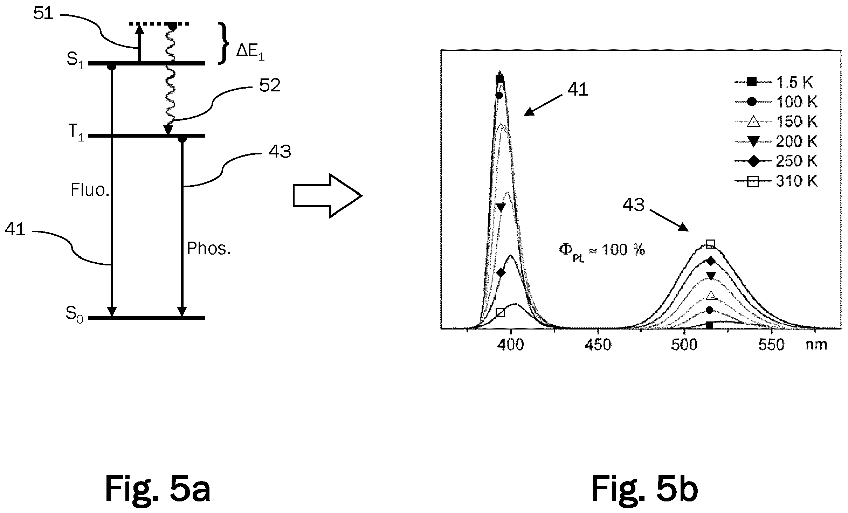

Die Figur 5a und 5b zeigen wie verschiedene Temperaturen auf das Lumineszenzverhalten Einfluss nehmen. Bei höheren Temperaturen nimmt die Fluoreszenz 41 ab, dafür die Phosphoreszenz 43 zu. Dies geschieht, weil bei höheren Temperaturen mehr thermische Energie E (= k_{B} T, wobei k_{B} die Bolzmannkonstante bezeichnet) bereit steht. Diese begünstigt die Spinumkehr.

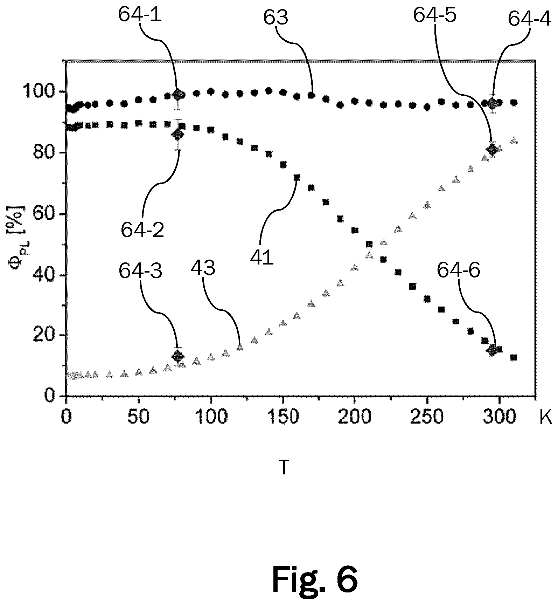

Die Figur 6 zeigt den in Figur 5b gezeigten Temperatureinfluss auf einen grünen und einen blauen Kanal. Wie bereits vorher erwähnt, wird bei der Phosphoreszenz ein Photon geringerer Energie freigesetzt, als bei der Fluoreszenz, so dass die Wellenlänge der Phosphoreszenz länger ist als diejenige der Fluoreszenz. In einer bevorzugten Ausführungsform des Sauerstoffsensors erfolgt die Fluoreszenz 41 im Bereich 375 - 445 nm. Dies entspricht violettem bis blauem Licht. Zur Vereinfachung wird im Folgenden dieses Spektrum schlicht blaues Licht genannt. In einer bevorzugten Ausführungsform des Sauerstoffsensors erfolgt die Phosphoreszenz 43 im Bereich 450 - 650 nm. Dies entspricht grünem bis gelbem Licht. Zur Vereinfachung wird im Folgenden dieses Spektrum schlicht grünes Licht genannt. Wie schon im Zusammenhang mit Fig. 5b erläutert, wird mit steigender Temperatur die Phosphoreszenz 43 gegenüber der Fluoreszenz 41 begünstigt. Daher steigt der Anteil des "grünen" Lichts vom gesamten emittierten Licht, während der Anteil des "blauen" Lichts zurückgeht. In der Fig. 6 sind auch Kalibrationspunkte 64-1 bis 64-6 dargestellt. So kann aus den gemessenen Lichtanteilen von "blauem" und "grünem" Licht die Temperatur bestimmt werden. Genauer kann man das Verhältnis der beiden Lichtanteile bilden und die Temperatur aus diesem Verhältnis und aus bekannten Kalibrationspunkten 64 extrapolieren.

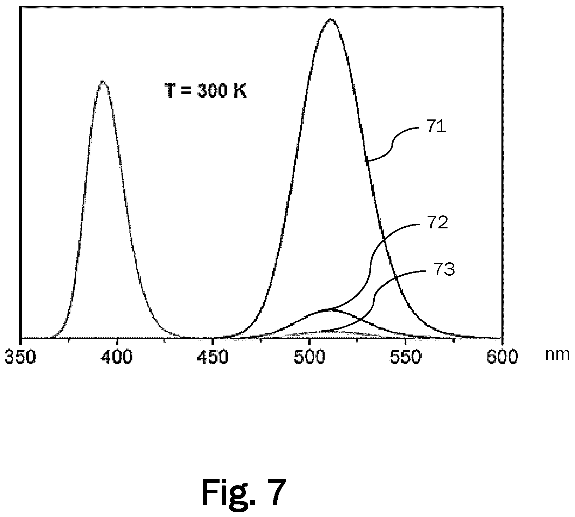

Die Figur 7 zeigt die Empfindlichkeit der Sauerstoffsensorik. Die y-Achse entspricht einer Intensität - genauer im konkreten Diagramm eine Intensität pro Wellenlänge. Die Fläche unter der Kurve ergibt die eigentliche Intensität, die in der Regel in Watt pro Quadratmeter gemessen wird. Die x-Achse entspricht der Wellenlänge in nm (Nanometer). Die Werte gelten bei einer Temperatur von 300 K.

Die Kurve links entspricht der Fluoreszenz / dem blauen Emissionslicht und ist auf Sauerstoff kaum empfindlich. Die Phosphoreszenz / das grüne Emissionslicht hingegen ist sehr sauerstoffempfindlich und wird bei Anwesenheit von Sauerstoff gelöscht. Die Kurve 71 entspricht einer Phosphoreszenz ohne Anwesenheit von Sauerstoff. Die Kurve 72 entspricht der Phosphoreszenz bei Anwesenheit von Luft, die bekanntlich auch (ca. 20,9 Vol.-%) Sauerstoff enthält. Die Kurve 73 entspricht der Phosphoreszenz bei Anwesenheit von reinem Sauerstoff.

Vorteile der vorliegenden Ergospirometrievorrichtung umfassen somit unter anderem, dass sie sehr sauerstoffempfindlich ist, dass sie einen sehr hohen dynamischen Bereich abdeckt, und dass deren Messtechnik über einen Intensitätsvergleich sehr einfach ist.

Die Auswertung kann über Intensität oder Wechselgrößen geschehen.

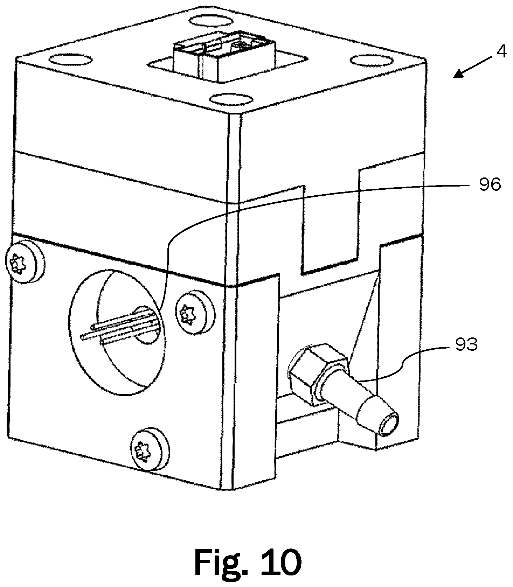

Die Messeinrichtung 4 (siehe Figur 10) kann dazu ausgebildet sein, die Lebensdauerauswertung über eine Detektion der Zerfallsdynamik, über eine schnelle Lebensdauerbestimmung, RLD, "Rapid Lifetime Determination", und/oder über eine Phasenmodulation vorzunehmen.

In einer zweiten Ausführungsform der vorliegenden Erfindung wird eine Ergospirometrievorrichtung mit einem Kohlenstoffdioxidsensor vorgestellt. Es sei explizit darauf hingewiesen, dass die zweite Ausführungsform auch mit der ersten Ausführungsform kombiniert werden kann, aber nicht muss.

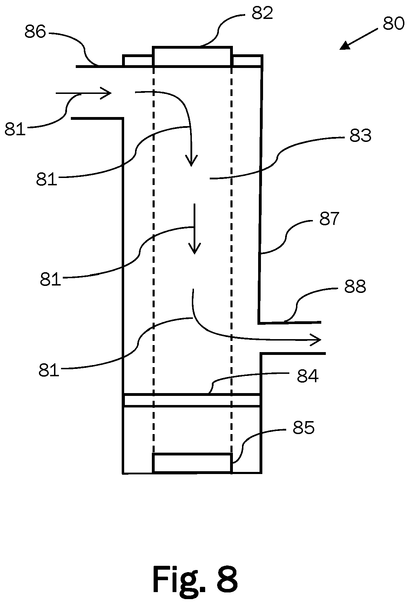

Die Figur 8 zeigt einen möglichen schematischen Aufbau eines Kohlenstoffdioxidsensors 80. Das Kohlendioxid ist in einem Gas enthalten, das über einen Eingang 86 in einen Hauptkörper 87 des Kohlendioxidsensors 80 geführt wird. Das Gas strömt dann durch den Hauptkörper 87 und durch einen Ausgang 88 wieder vom Hauptkörper 87 hinaus. Die Zu- und Abfuhr beim Eingang 86 bzw. beim Ausgang 88 kann über einen Schlauch 93 erfolgen. Der Weg des Gases ist durch die Pfeile 81 dargestellt. An einem Ende des Hauptkörpers des Kohlendioxidsensors 80 ist eine Strahlungsquelle 82 angebracht, wobei mit Strahlung eine elektromagnetische Strahlung gemeint ist. Vorzugsweise ist diese Strahlungsquelle 82 eine Leuchtdiode 82 (auch LED genannt). Vorzugsweise kann die Leuchtdiode eine MIR-LED 82, für einen Wellenlängenbereich im mittleren Infrarotbereich aufweisen. Insbesondere kann dieser Wellenlängenbereich zwischen 3 µm und 8 µm und besonders vorzugsweise zwischen 4,1 µm und 4,4 µm liegen. Vorzugsweise kann die Leuchtdiode für einen Wellenlängenbereich im mittleren Infrarot derart modulierbar sein, dass Taktzeiten von mindestens 1 kHz, bevorzugt aber nicht zwingend 100 kHz oder mehr, erreicht werden. Damit werden schnelle Ansprechzeiten für den Kohlendioxidsensor 80 möglich. Vorzugsweise kann die Leuchtdiode eine MIR-LED mit einem Reflektor aufweisen, so dass eine sehr hohe Strahlqualität erzielt werden kann.

Diese Strahlungsquelle 82 beleuchtet das Innere des Hauptkörpers 87, durch den das Gas strömt. Der Strahl 83 der Strahlungsquelle 82 trifft am anderen Ende auf ein Sensorelement 85. Dieses Sensorelement kann vorzugsweise ein Photodetektor, insbesondere ein Quantentopf-Infrarot-Photodetektor (Quantum Well Infrared Photodetector, QWIP) sein. Vorzugsweise kann der Photodetektor zweikanalig ausführt sein, so dass eine Auswertung eines Referenzsignals zur Überwachung des Strahlers ermöglicht wird. Zudem kann über die zwei Kanäle Alterungsprozessen der Strahlungsquelle 82, insbesondere im Falle, dass die Strahlungsquelle 82 eine LED ist, entgegenwirken. Zudem kann über die zwei Kanäle eine Beeinträchtigung durch Verschmutzungen entgegenwirkt werden. Das Erfassen der Kohlendioxidkonzentration im Atemgas kann auf einem Erfassungsprinzip eines nichtdispersiven Infrarotsensors, NDIR-Prinzip, basieren. Damit können schnelle Ansprechzeiten für den Kohlendioxidsensor 80 realisiert werden. Entsprechend kann ein Filter 84 in den Strahlengang zwischen der Strahlenquelle und dem Sensorelement 85 eingebaut sein. Der Filter 84 kann dann die für die Kohlendioxiderfassung relevante Strahlung durchlassen und andere Wellenlängen wegfiltern. Die relevante Strahlung von Kohlendioxid kann eine Wellenlänge bei 4,3 µm sein. Die Strahlung von der Strahlungsquelle 82 kommt bei der relevanten Wellenlänge bis zum Sensorelement 85 durch, wenn sich kein Gas (oder zumindest kein Kohlendioxid) im Innern des Hauptkörpers 87 des Kohlendioxidsensors 80 befindet. Ist jedoch Kohlendioxid vorhanden kommt mit zunehmender Konzentration immer weniger Strahlung durch. Messungen ergaben, dass bei 5000 ppm auf eine Rohrlänge von 200 mm das Signal um 26% abgenommen hat

Ein Vorteil dieses Kohlendioxidsensors 80 ist eine sehr kurze Ansprechzeit. Insbesondere kann so eine Ansprechzeit von 0,1 s oder weniger erreicht werden.

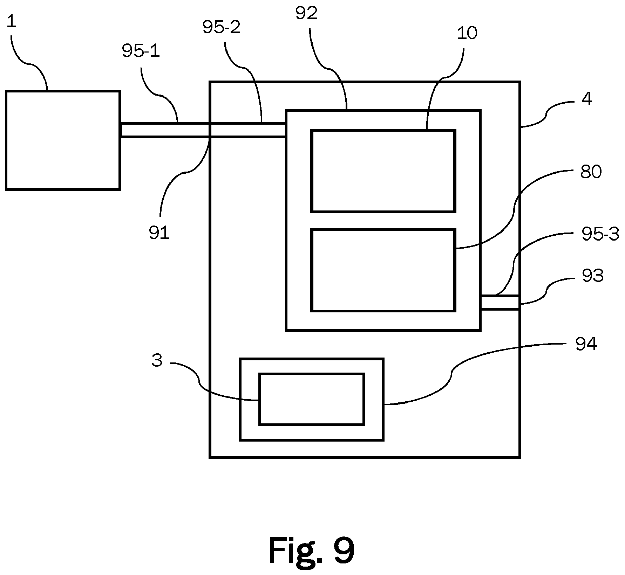

Die Figur 9. zeigt schematisch ein pneumatisches Konzept des Gesamtsystems. Ein Grundkörper 1, der eine Atemmaske oder ein Mundstück aufweist, kann über einen Schlauch 95-1 mit einer Messeinrichtung 4 verbunden sein. Die Messeinrichtung 4 weist unter anderem eine Recheneinheit 3 zum Verarbeiten der erfassten Parameter des Atemgases auf. Für das pneumatische Konzept ist die Recheneinheit 3 jedoch nicht relevant. Daher kann die Recheneinheit 3 in einem Bereich 94 untergebracht sein, der zumindest pneumatisch von der Messkammer 92 der Messeinrichtung 4 getrennt ist. Optionalerweise kann die Recheneinheit 3 auch von der Messeinrichtung 4 räumlich getrennt und nur elektrisch verbunden sein. Alternativ dazu kann die Recheneinheit 3 in einem Bereich 94 der Messeinrichtung 4 untergebracht sein. In dem Bereich 94 kann auch weitere Elektronik, z. B. eine Platine zur Anregung und Auswertung des Sensorspots 13 untergebracht sein. Optionalerweise kann die pneumatische Anbindung des Grundkörpers 1 an die Messeinrichtung 4 über einen Schlauch 95-1 erfolgen. Insbesondere kann der Schlauch 95-1 an einen Eingang 91 der Messeinrichtung 4 andocken. Des Weiteren kann die pneumatische Anbindung innerhalb der Messeinrichtung 4 derart ausgestaltet sein, dass die Messeinrichtung 4 eine Messkammer 92 aufweist, die mit dem Eingang 91 pneumatisch gekoppelt ist. Diese pneumatische Kopplung kann optionalerweise über einen Schlauch 95-2 erfolgen. Ein Messvolumen kann durch die Messkammer 92 festgelegt sein. Umgekehrt kann ein erstrebtes Messvolumen die Dimensionierung der Messkammer 92 beeinflussen. Insbesondere kann die Messkammer 92 für ein Messvolumen von Bruchteilen von einem Milliliter (ml) bis wenige Milliliter für eine korrekte Messung ausgelegt sein. Vorzugsweise kann die Messkammer 92 für ein Messvolumen von kleiner gleich 0,5 Milliliter, insbesondere zwischen 0,1 und 0,5 Milliliter ausgelegt sein. Zumindest Teile des Sensors 10, 80 können in oder an (also am Rande der) der Messkammer 92 untergebracht sein. Die Messkammer 92 kann pneumatisch mit einem Ausgang 93 der Messeinrichtung 4 gekoppelt sein. Diese Kopplung kann optionalerweise über einen Schlauch 95-3 erfolgen.

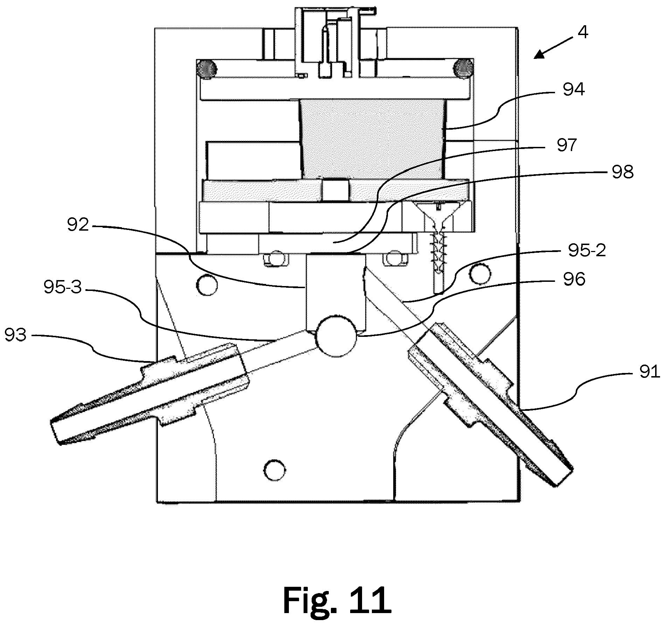

Die Figuren 10 und 11 zeigen eine Ausführungsform der Messeinrichtung 4 der beanspruchten Ergospirometrievorrichtung. Dabei ist die Figur 10 eine perspektivische Darstellung der Messeinrichtung 4 und die Figur 11 eine Schnittdarstellung der Messeinrichtung 4. Man beachte die Position des Ausgangs 93 damit dem Leser die relative Orientierung der beiden Darstellungen klar ist. Wie schon weiter oben erklärt können Teile des Sensors 10, 80 in die Messkammer 92 der Messeinrichtung 4 untergebracht sein. Bevorzugt aber nicht zwingend können Teile sowohl eines Sauerstoffsensors 10 als auch eines Kohlendioxidsensor 80 in einer einzigen Messkammer untergebracht sein. Die gezeigte Ausführungsform enthält eine Aussparung 96, damit dadurch ein Teil des Sensors durch diese in die Messkammer 92 ragen kann. Insbesondere kann ein Teil des Kohlendioxidsensors 80 durch die Aussparung 96 in die Messkammer 92 eingebracht werden. Alternativ oder zusätzlich kann am Rande der Messkammer 92 ein Teil 97 des Sensors 10, 80 untergebracht sein. Insbesondere kann sich am Rande der Messkammer 92 eine Sensorschicht 98 / eine aktive Fläche des Sauerstoffsensors 10 / Spot 13 (als Teil 97 des Sauerstoffsensors 10) befinden. Eine Atemgasführung, wie z. B. ein Schlauch 95-2, von einem Eingang 91 der Messeinrichtung 4 zum Sensor 10 kann derart ausgerichtet sein, dass das Atemgas direkt auf die Sensorschicht 98 / die aktive Fläche des Spots 13 geblasen wird. Für eine für einen Kohlendioxidsensor 80 angemessenere Absorptionsstrecke kann der Ausgang 93 der Messeinrichtung 4 im Vergleich zum Eingang 91 der Messeinrichtung 4 versetzt, insbesondere in der Ebene versetzt, sein.

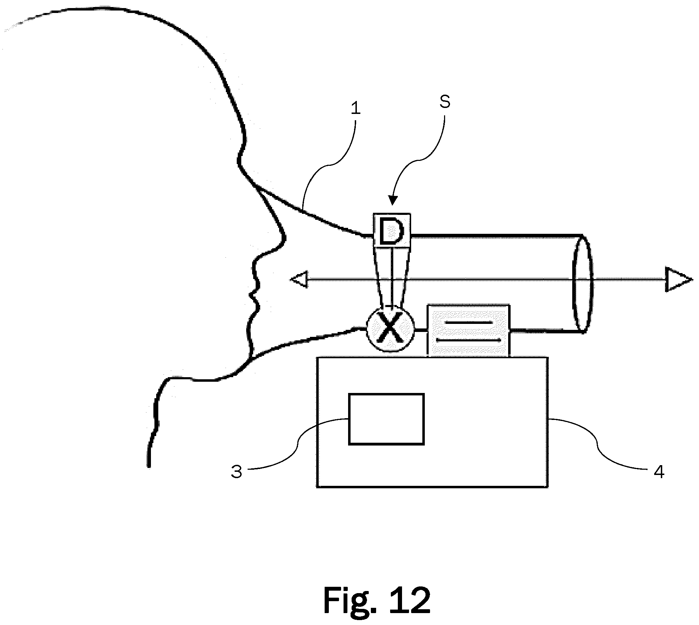

Die Figur 12 zeigt einen schematischen Aufbau einer Ergospirometrievorrichtung des Stands der Technik. Sie weist einen Grundkörper 1 und eine Messeinrichtung 4 auf, die sich allerdings von der Messeinrichtung 4 der vorliegend beanspruchten Ergospirometrievorrichtung unterscheidet.

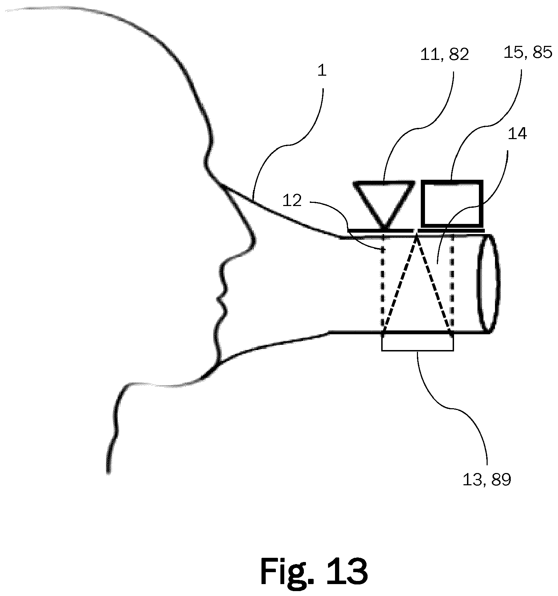

Die Figur 13 zeigt einen vorteilhaften Aufbau einer erfindungsgemäßen Ergospirometrievorrichtung in einer sehr vorteilhaften Ausführungsform. An einem Grundkörper 1 wird direkt (in der Darstellung oben links) eine Lichtquelle 12 oder eine Strahlquelle 82 befestigt sein. So kann an der gegenüberliegenden Seite auch direkt am Grundkörper der Sensorspot 13 im Fall des Sauerstoffsensors 10 bzw. ein Reflektor 89 im Fall des Kohlendioxidsensors angebracht sein. Auf wieder der ersten Seite des Grundkörpers kann dann wieder das Sensorelement 15 bzw. das Sensorelement 85 angebracht sein. Der Grundkörper 1 kann zumindest an diesem Abschnitt transparent und nichtfluoreszierend sein. So ist der Sauerstoffsensor 10 (siehe Fig. 1) bzw. der Kohlendioxidsesor 80 der Messeinrichtung direkt mit dem Grundkörper 1 verbunden. Dies ermöglicht eine sehr kompakte Bauweise. Insbesondere können eine Lichtquelle 11 bzw. eine Strahlquelle 82 direkt an dem Grundkörper angebracht sein, womit eine unerwünschte Verschmutzung erschwert wird. Zudem können ein Sensorelement 15 bzw. 85 direkt an dem Grundkörper angebracht sein, womit eine unerwünschte Verschmutzung (weiter) erschwert wird. Damit Sauerstoff in die Sensorschicht eindiffundieren kann, kann sich das Sensorelement derart angebracht sein, dass das Sensorelement mit dem Atemgas Kontakt hat. Alternativ dazu kann auch der Spot an einen transparenten Grundkörper eingeschweißt sein, womit auch eine unerwünschte Verschmutzung des Spots erschwert wird.

### Herstellungsverfahren

Ein weiterer Aspekt der vorliegenden Erfindung ist die Herstellung einer Ergospirometrievorrichtung wie oben beschrieben. Das Verfahren weist folgende Schritte auf:
- Bereitstellen einer Messeinrichtung 4 zum Erfassen von Parametern des Atemgases, die eine Recheneinheit 3 zum Verarbeiten der erfassten Parameter des Atemgases aufweist,
- Bereitstellen eines Grundkörpers 1, der eine Atemmaske oder ein Mundstück aufweist, und
- Verbinden der Messeinrichtung 4 mit dem Grundkörper 1.

Die bereitgestellte Messeinrichtung 4 kann dabei Eigenschaften aufweisen, die oben beschrieben sind. Der bereitgestellt Grundkörpers 1 kann dabei Eigenschaften aufweisen, die oben beschrieben sind. Insbesondere kann ein Sensorspot mit Kupfer- oder Platin-Metallkomplexen mit einem Polymer beschichtet werden, indem das Polymer lediglich flüssig aufgetragen und trocknengelassen wird. So kann eine dünne Schicht aufgetragen werden. Ein Vorteil davon ist, dass das Verfahren günstig ist.

Ein Vorteil der vorliegenden Ergospirometrievorrichtung ist, dass sie selbstreferenzierend ist und dass sie eine sehr hohe Sauerstoff-Empfindlichkeit aufweist (bis in den ppb-Bereich ("parts per billion", also in Teile pro Milliarde)). Es kann zugleich einen hohen dynamischen Bereich (optional bis 9 Größenordnungen) abdecken. Zudem ist auch die Emissionsquantenausbeute hoch. Der Metallkomplex kann ein Kupfer-Metallkomplex sein. Dieses Material ist zum einen preiswert und zum anderen leicht verfügbar. Zudem ist Kupfer (wahrscheinlich) nicht toxisch. Ein weiterer Vorteil ist, dass die Emissionen spektral voneinander getrennt sind. Dies vereinfacht die Messtechnik weiter und erlaubt einen Intensitätsvergleich. Es ist zudem anzumerken, dass mit der vorliegenden Ergospirometrievorrichtung eine außerordentlich hohe Quantenausbeute erzielt werden kann. Mit der sehr schnellen Auswertung werden die Anforderungen für ein Breath-by-Breath-Prinzip mit Leichtigkeit erfüllt.

## Patentansprüche

1. Ergospirometrievorrichtung zur Erfassung von Parametern eines Atemgases, mit
einem Grundkörper (1), der eine Atemmaske oder ein Mundstück aufweist und einen Atemgasführungsabschnitt (5) aufweist, und
einer Messeinrichtung (4) zum Erfassen von Parametern des Atemgases, die eine Recheneinheit (3) zum Verarbeiten der erfassten Parameter des Atemgases aufweist,
wobei die Parameter des Atemgases zumindest eine Sauerstoffkonzentration und/oder eine Kohlenstoffdioxidkonzentration im Atemgas umfassen,
wobei die Messeinrichtung (4) zumindest einen optischen Sauerstoffsensor (10) zum Erfassen der Sauerstoffkonzentration des Atemgases und/oder einen optischen Kohlenstoffdioxidsensor aufweist, und
wobei das Erfassen der Sauerstoffkonzentration auf einem Prinzip der Lumineszenzlöschung und/oder einem Prinzip der dualen Emission beruht.

2. Ergospirometrievorrichtung nach Anspruch 1, wobei die Messeinrichtung (4) dazu ausgebildet ist, die Erfassung der Sauerstoffkonzentration über eine Lebensdauerauswertung vorzunehmen,
wobei optional die Messeinrichtung (4) dazu ausgebildet ist, die Lebensdauerauswertung über eine Detektion der Zerfallsdynamik, über eine schnelle Lebensdauerbestimmung, RLD, "Rapid Lifetime Determination", und/oder über eine Phasenmodulation vorzunehmen.

3. Ergospirometrievorrichtung nach Anspruch 1 oder 2, wobei die Messeinrichtung (4) dazu ausgebildet ist, die Erfassung der Sauerstoffkonzentration über eine Auswertung der Quantenausbeute und/oder der Intensität vorzunehmen, und/oder
wobei die Messeinrichtung (4) zur ratiometrischen Bestimmung der Sauerstoffkonzentration eingerichtet ist, aus einem Intensitätsverhältnis der Fluoreszenz und Phosphoreszenz die Sauerstoffkonzentration zu bestimmen.

4. Ergospirometrievorrichtung nach zumindest einem der vorhergehenden Ansprüche, wobei der Sauerstoffsensor (10) auf dem Prinzip der Lumineszenzlöschung mit Metall-Komplexen - optional Übergangsmetall-Komplexen - und/oder Polymeren arbeitet und wobei die Übergangsmetall-Komplexe bevorzugt Platin und/oder Kupfer umfassen, und/oder
wobei über Variation von Metall-Komplex und Anregungsenergie eine Duale Emission möglich ist, bevorzugt in den Bereichen 375 - 445 nm und 450 - 650 nm, um Informationen über Druck und/oder Temperatur abzuleiten.

5. Ergospirometrievorrichtung nach zumindest einem der vorhergehenden Ansprüche, wobei der Sensor auch über Feststoffe funktioniert, die nicht fluoreszierend sind und die transparent sind, insbesondere Glas, Polycarbonat, PMMA, PET.

6. Ergospirometrievorrichtung nach zumindest einem der vorhergehenden Ansprüche, wobei der Sauerstoffsensor (10) temperatur- und barometrisch kompensiert ist und bevorzugt einen integrierten Drucksensor umfasst, und/oder
wobei die Sensorvorrichtung selbstreferenzierend ist, und/oder
wobei die Ergospirometrievorrichtung eine mobile Ergospirometrievorrichtung ist.

7. Ergospirometrievorrichtung nach zumindest einem der vorhergehenden Ansprüche, wobei die Messeinrichtung (4) eine Ansprechzeit von kleiner oder gleich 0,1 s aufweist und/oder
wobei eine Sensorschicht des Sauerstoffsensors (10) eine Diffusionskonstante von geringer als 5 Sekunden, und besonders bevorzugt geringer als 0,1 s aufweist, und/oder
wobei ein Trägermaterial der Sensorschicht eine transparente Polyesterschicht oder Polyesterfolie umfasst, welche bevorzugt eine Schichtdicke von weniger als 800 µm aufweist, und wobei die Sensorschicht bevorzugt ein Sensorspot zur kontaktlosen Messung ist.

8. Ergospirometrievorrichtung nach zumindest einem der vorhergehenden Ansprüche, wobei ein Anregungslicht der Anregungsdiode des Sauerstoffsensors (10) mit einer Anregungsfrequenz von mehr als 1 Hz - optional zwischen 10 Hz und 100 kHz - blinkt und das von der Sensorschicht, insbesondere dem Sensorspot, emittierte Licht im Bereich von 450 - 650 nm und/oder 375 - 445 nm liegt.

9. Ergospirometrievorrichtung nach zumindest einem der vorhergehenden Ansprüche, wobei die Messeinrichtung (4) am Grundkörper (1) bereitgestellt ist, damit die Parameter direkt in einem Atemgasstrom erfassbar sind, der durch den Grundkörper (1) geführt wird, sodass eine Erfassung der Parameter des Atemgases in situ ermöglicht wird.

10. Ergospirometrievorrichtung nach zumindest einem der vorhergehenden Ansprüche, wobei zumindest Teile der Messeinrichtung (4) von dem Grundkörper (1) beabstandet sind, sodass eine Erfassung der Parameter des Atemgases in Extraktion ermöglicht wird.

11. Ergospirometrievorrichtung nach zumindest einem der vorhergehenden Ansprüche, wobei der Kohlenstoffdioxidsensor zum Erfassen einer Kohlendioxidkonzentration im Atemgas vorgesehen ist, wobei das Erfassen der Kohlendioxidkonzentration im Atemgas auf einem Erfassungsprinzip eines nichtdispersiven Infrarotsensors, NDIR-Prinzip, basiert.

12. Ergospirometrievorrichtung zumindest einem der vorhergehenden Ansprüche, wobei die der Kohlenstoffdioxidsensor einen Photodetektor und eine Leuchtdiode, insbesondere MIR-LED, für einen Wellenlängenbereich im mittleren Infrarotbereich aufweist, insbesondere im Wellenlängenbereich zwischen 3 µm und 8 µm und besonders vorzugsweise zwischen 4,1 µm und 4,4 µm,
wobei optional die Leuchtdiode für einen Wellenlängenbereich im mittleren Infrarot derart modulierbar ist, dass Taktzeiten von mindestens 1 kHz erreicht werden, und/oder
wobei optional der Photodetektor ein Quantentopf-Infrarot-Photodetektor (Quantum Well Infrared Photodetector, QWIP) ist, und/oder
wobei optional die Leuchtdiode eine MIR-LED mit einem Reflektor aufweist, so dass eine sehr hohe Strahlqualität erzielt wird, und/oder
wobei optional der Photodetektor zweikanalig ausgeführt ist, so dass eine Auswertung eines Referenzsignals zur Überwachung des Strahlers ermöglicht wird.

13. Ergospirometrievorrichtung nach zumindest einem der vorhergehenden Ansprüche, wobei der Grundkörper (1) den Atemgasführungsabschnitt (5) zur Führung des Atemgasstroms mit einem probandenseitigen Atemgaseinlass und einem Atemgasauslass aufweist und wobei der Sauerstoffsensor (10) im Atemgasführungsabschnitt vorgesehen ist.

14. Ergospirometrievorrichtung nach zumindest einem der vorhergehenden Ansprüche, wobei die Ergospirometrievorrichtung zumindest in Teilen, vorzugsweise in Gänze auf Kondensation resistent ist, so dass die Ergospirometrievorrichtung fähig ist, zumindest ein Teil der Parameter des Atemgases - insbesondere eine Sauerstoffkonzentration des Atemgases - auch bei hoher Feuchte oder mit kondensierendem Wasser in der Messkammer zu erfassen, und/oder
wobei die Messeinrichtung (4) eine Empfindlichkeit bis wenige ppb aufweist und/oder einen sehr hohen Dynamikumfang bis 100%, bis zu 9 Größenordnungen aufweist.

15. Verfahren zum Herstellen einer Ergospirometrievorrichtung , wobei das Verfahren folgende Schritte aufweist:
Bereitstellen einer Messeinrichtung (4) zum Erfassen von Parametern des Atemgases, die eine Recheneinheit (3) zum Verarbeiten der erfassten Parameter des Atemgases aufweist,
Bereitstellen eines Grundkörpers (1), der eine Atemmaske oder ein Mundstück aufweist, und
Verbinden der Messeinrichtung (4) mit dem Grundkörper (1).
